(19) Europäisches Patentamt
European Patent Office
Office européen des brevets

(11) EP 0 433 679 B1

(12) **EUROPEAN PATENT SPECIFICATION**

(45) Date of publication and mention of the grant of the patent: **02.05.1997 Bulletin 1997/18**

(51) Int. Cl.$^6$: **A61K 31/535**, A61K 31/47

(21) Application number: **90122096.2**

(22) Date of filing: **19.11.1990**

# (54) Pharmaceutical compositions for inhibition of maillard's reaction

Pharmazeutische Zusammensetzungen zur Hemmung der Maillardreaktion

Compositions pharmaceutiques pour l'inhibition de la réaction de maillard

(84) Designated Contracting States:
**AT BE CH DE DK ES FR GB GR IT LI LU NL SE**

(30) Priority: **20.11.1989 JP 301413/89**

(43) Date of publication of application:
**26.06.1991 Bulletin 1991/26**

(73) Proprietor: **Senju Pharmaceutical Co., Ltd.**
**Osaka-shi, Osaka 541 (JP)**

(72) Inventor: **Inoue, Jun**
**Settsu-shi, Osaka-fu (JP)**

(74) Representative: **Kinzebach, Werner, Dr. et al**
**Patentanwälte**
**Reitstötter, Kinzebach und Partner**
**Postfach 86 06 49**
**81633 München (DE)**

(56) References cited:
**WO-A-90/01558**
**DE-A- 3 904 731**
**US-A- 4 359 455**
**US-A- 4 861 775**

- **DATABASE WPIL, accession no. 81-52731D, Derwent Publications Ltd, London, GB; & JP-A-56 065 874 (MITSUI TOATSU CHEM. INC.)**
- **CHEMICAL ABSTRACTS, vol. 96, no. 20, 17th May 1982, page 402, abstract no. 168726x, Columbus, Ohio, US; & JP-A-81 164 112 (S. SHIOZU) 17-12-1981**
- **Toxicology and Applied Pharmacology Volume 44,Number 2,1978,pages 225-249**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Rank Xerox (UK) Business Services
2.14.3/3.4

## Description

BACKGROUND OF THE INVENTION

This invention relates to the inhibition of denaturation reaction of proteins by glucose (Maillard's reaction.) More specifically this invention relates to the inhibition of the formation of Amadori rearrangement products which originate from non-enzymatic bond formation between glucose and proteins.

The reaction in which proteins turn brown by reacting non-enzymatically with reductive sugars such as glucose was first reported by Maillard in 1912 [Maillard, L.C., Compt. Rend. Soc. Biol., 72:599 (1912).] Since then, the reaction has been widely recognized by the name of Maillard's reaction in the field of food chemistry. For example, it has been noted that proteins react with glucose in stored or heated food, generate a brown color and finally are denaturated by formation of cross-linkings among molecules.

Later, attentions came to be attracted to reactions of glucose with proteins which may occur in living bodies when Rahbar reported that the level of $Hb_{A1c}$, a minor component of hemoglobin, was found elevated in red blood cells of diabetic patients [Rahbar, S., Clin. Chim. Acta, 22:296 (1968).] And, through structural analysis of $Hb_{A1c}$, it has been confirmed that Maillard's reaction occurs in living bodies.

The mechanism of Maillard's reaction in living bodies has been presented by Brownlee et al. [Brownlee, M. et al., Science, 232:1629 (1986).] The reaction proceeds as follows.

At first, the aldehyde group of the open-ring structure of glucose reacts with an amino group in protein molecule to form a schiff's base. The resulting schiff's base is unstable and is rapidly converted into Amadori rearrangement product via intra-molecular rearrangement reaction. If this protein is maintained for a long period within the body, the rearranged product undergoes a gradual dehydration reaction to form a new glucose derivative. This derivative then irreversively forms cross-linkings with a variety of molecules including proteins to form bridges among molecules, thus yielding aggregation products of, chiefly, proteins.

This type of product resulting from advanced reactions of glycosylated proteins is usually abbreviated to AGE (Advanced Glycosylation End product.)

In parallel to the formation of AGE, biological adaptibility of the protein is lowered, and the protein becomes less soluble and more resistant to proteases and, in many cases, turns to yellow-brown and fluorescent.

Though observed also in healthy human, Maillard's reaction is markedly noted in those with diabetes mellitus, which is characterized by the elevation of blood glucose. Maillard's reaction is especially notable in proteins with slower rate of metabolic turnover, for example crystallins, which are the structural proteins in the lens, and collagens. While a variety of disorders, for example neuropathy, cataract, nephropathy, retinopathy, arthrosclerosis and atherosclerosis, are noted as complications of diabetes mellitus, these disorders bear a very close resemblance with disorders noted quite frequently in aged human.

It, therefore, is regarded that AGE is also formed gradually from proteins with a slower turnover rate by glycosylation with glucose even under a nomal level of blood sugar.

Upon the said background, efforts have been made in search of compounds which may inhibit Maillard's reaction within living bodies. An example of such efforts has been shown by Brownlee as cited who reported that aminoguanidine inhibits Maillard's reaction in vitro and suppresses AGE formation in arterial walls of diabetic rats in vivo. In Japanese Patent Publication Kokai No. 142114/87, it has been suggested that aminoguanidine, α-hydrazinohistidine and lysine may block the active carbonyl group of Amadori rearrangement products to inhibit AGE formation. It has also been disclosed that different compounds may suppress Maillard's reaction. Such compounds include thiosemicarbazides, 1,3-diaminoguanidine and benzoylhydrazine (Japanese Patent Publication Kokai No. 56614/89), and various derivatives of guanidine (Japanese Patent Publication Kokai No. 83059/89.)

In the patent publications cited above, researches for inhibitors of Maillard's reaction were made using the amount of AGE, the end product of Maillard's reaction, as an index. The present inventor, instead, took the inhibition of formation of Amadori rearrangement product as an index in the investigation. This was based on an estimation that a markedly effective inhibition of Maillard's reaction may be expected by inhibiting the very formation of Amadori rearrangement product, which is the immediate causing factor in protein aggregation process in Maillard's reaction.

Bruggemann et al. [J. Bruggemann et al., Lebensm. Unters. Forsch., 137:137-143 (1968)] and Finot et al. [P.A. Finot et al., Experientia, 24:1097-1099 (1968)] have reported that the amount of ε-N-(furoyl-methyl)-L-lysine hereinafter referred to as "furosine", which is an Amadori rearrangement product resulted from non-enzymatic glycosylation of ε-amino residue of lysine in proteins, may be taken as an index of the non-enzymatic glycosylation of protein molecules. The present inventor made an intensive research for the optimal experimental condition for formation of furosine from protein dissolved in water containing glucose, and, according to the condition thus established, evaluated various compounds for the presence and strength of inhibitory effect on furosine formation.

In US 4,359,455 a test composition for evaluating dental caries activity is disclosed, comprising resazurin in admixture with a saccharide selected from the group consisting of sucrose, glucose and fructose.

G.A. Lutty discloses in Toxicology and Applied Pharmacology (1978), 44, 225-249, the use of resazurin and other

dyes in certain angiographic techniques. In order to determine the acute intravenous toxicity resazurin was dissolved in distilled water and injected in the caudal vein of mice.

As a result, the present inventor discovered that some of the quinolinequinone derivatives have a potent inhibitory effect. Based on the discovery, evaluation was continued, which lead to the findings that a certain class of known compounds have comparably potent inhibitory effects on furosine formation.

Such compounds include quinolinequinones with various substitutional groups, reduced type compounds thereof and other compounds which may be derived from quinone or quinolinequinone. The present invention has been accomplished upon these findings.

## SUMMARY OF THE INVENTION

It, therefore, is a principal object of the present invention to provide pharmaceutical compositions as inhibitors of Maillard's reaction in human body.

Another principal object relates to the use of compounds of formulae (I) to (IV) for preparing a pharmaceutical composition for the treatment and prophylaxis of disorders in human body which may develop via Maillard's reaction. Such disorders include diabetic complications, for example coronary heart disease, peripheral circulation disorders, cerebrovascular disorders, neuropathy, nephropathy, arteriosclerosis, arthrosclerosis, cataract and retinopathy, and age-associated disorders such as atherosclerosis, coronary heart disease, cerebrovascular disorders and senile cataract.

Thus, the pharmaceutical composition of the present invention comprises in admixture with a pharmaceutically acceptable carrier;

(a) a 4-hydroxy-5,8-dioxoquinoline derivative of the formula (I) or a pharmaceutically acceptable salt thereof:

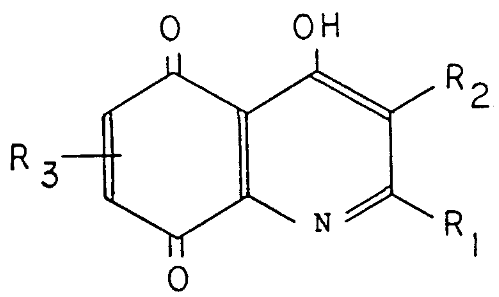

(I)

wherein $R_1$ and $R_2$ are each hydrogen, methyl, trifluoromethyl, carboxy, methoxycarbonyl or ethoxycarbonyl, and $R_3$ is hydrogen or hydroxy;

(b) a 4,5,8-trihydroxyquinoline derivative of the formula (II) or a pharmaceutically acceptable salt thereof:

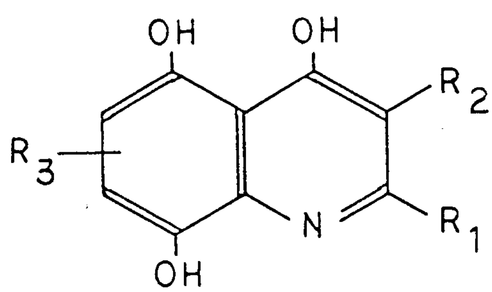

(I)

wherein $R_1$, $R_2$ and $R_3$ are as defined hereinbefore;

(c) a 3-oxophenoxazine derivative of the formula (III) or a pharmaceutically acceptable salt thereof:

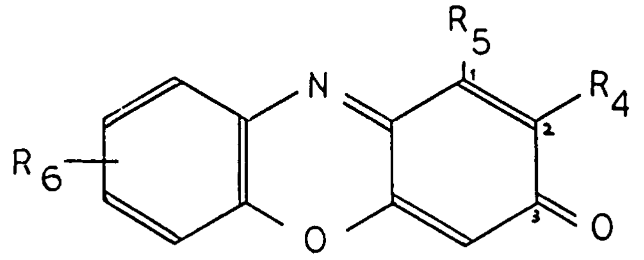

(III)

wherein $R_4$ and $R_5$ are each hydrogen or form by incorporation of $C_1$ and $C_2$ carbon atoms a condensed [2,1-b] pyridine ring optionally substituted with a hydroxyl group and/or a carboxyl group, and $R_6$ is hydrogen or hydroxy; or (d) a 3-oxophenoxazine N-oxide of the formula (IV) or a pharmaceutically acceptable salt thereof:

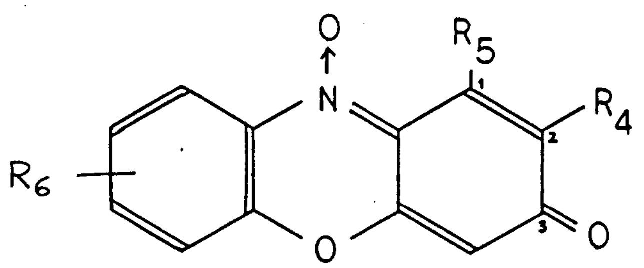

(IV)

wherein $R_4$, $R_5$ and $R_6$ are as defined hereinbefore; with the proviso that resazurin is not applied.

DETAILED DISCUSSION

The examples of pharmaceutically acceptable salts or the compounds described hereinbefore by the formulas (I) to (IV) include, in particular, alkali metal salts thereof such as sodium salt and potassium salt, alkaline earth metal salts thereof such as calcium salt and magnesium salt, and salts thereof with inorganic acids such as hydrochloric acid, sulfuric acid and phosphoric acid, or with organic acids such as acetic acid and maleic acid.

Salts which are acceptable as pharmaceuticals are included in the scope of the present invention.

The Maillard's reaction inhibitors of the present invention may be used for the treatment or prophylaxis of a variety of disorders in human body mentioned hereinbefore. For this purpose, the inhibitors of Maillard's reaction of the present invention may be administered orally or parenterally. The inhibitors may also be applied topically, for example, in the form of eye drops.

The Maillard's reaction inhibitor represented by the formulas (I) to (IV) (also in the form of pharmaceutically acceptable salts thereof) may be administered orally at a dose of 1 to 1,000 mg/day, more preferably 5 to 200 mg/day. For injection, the dose may be 0.1 to 100 mg/day, more preferably 1 to 50 mg/day.

For topical application to the eye, the Maillard's reaction inhibitor of the present invention may be applied in the form of eye drops containing the inhibitor at a concentration of 0.05 to 5.0 w/v %, more preferably 0.1 to 2.0 w/v %.

A suitable dose may be set according to the type and severity of disorders and schedules of treatment in each case.

The Maillard's reaction inhibitor represented by the formulas (I) to (IV) or pharmaceutically acceptable salt thereof may be formed into, for example, tablets, pilles, powder, granules or capsules for oral administration, aqueous or non-aqueous solution, suspension or emulsion for injection, or eye drops or eye ointment for ophthalmic topical use.

For preparing pharmaceutical composition of the present invention into the form of tablets, ingredients usually incorporated in tablet preparation may suitably be utilized.

Such ingredients include, for example, diluent bases such as hydroxypropylcellulose, crystalline cellulose, corn starch, polyvinylpyrrolidone, and magnesium metasilicate aluminate, lubricants such as magnesium stearate, disintegrators such as fibrinous calcium gluconate, and solubilizers such as glutamic acid and aspartic acid.

For preparing a pharmaceutical composition of the present invention into the form of aqueous injection, ingredients usually incorporated in injectable preparations may suitably be utilized. Such ingredients include, for example, buffering agents such as phosphates, preservatives such as chlorobutanol, stabilizers such as sodium sulfite, and isotonizers such as sodium chloride.

For preparing a pharmaceutical composition of the present invention into the form of eye drops, ingredients usually

incoporated in the formation of eye drops may suitably utilized. Such ingredients include, for example, buffering agents such as phosphates, borates and acetates, preservatives such as chlorobutanol and benzalkonium chloride, stabilizers such as sodium sulfite and sodium edetate, isotonizers such as sodium chloride, potassium chloride and glycerol, and solubilizers such as polysorbate 80 and cyclodextrins.

The inhibitors of Maillard's reaction represented by the formulas (I) to (IV) and pharmaceutically acceptable salts thereof, inhibit the very formation of Amadori rearrangement product, the immediate causing factor of cross linkings among protein molecules.

The pharmaceutical compositions of the present invention, accordingly, may be useful for treatment and prophylaxis of diabetic complications, for example coronary heart disease, peripheral circulation disorders, cerebrovascular disorders, neuropathy, nephropathy, arteriosclerosis, arthrosclerosis, cataract and retinopathy, and age-associated disorders such as atherosclerosis, coronary heart disease, cerebrovascular disorders and senile cataract.

The effect of the Maillard's reaction inhibitors of the present invention was determined as follows.

Pharmacological Test

Test compounds:

Compounds T-1 to T-11 as described in Table 1 were tested. They were known compounds and obtained as follows.

T-1 was obtained by hydrogen peroxide oxidation, in alkaline aqueous solution, of 4,5,6,8-tetrahydroxyquinoline which had been prepared according to the specification of the Japanese Patent Publication No. 2269/60.

T-2 and T-9 were prepared from 2,5-dimethoxyaniline according to the method described by G.S. Bajwa et al. [Journal of Medicinal Chemstry, 16:134 (1973).]

T-3 was prepared by oxidation of T-2 by a conventional method.

T-4, T-5 and T-6 were prepared by oxidation, hydrolysis and oxidation after hydrolysis, respectively, of 4,5,8-trihydroxy-2-methoxycarbonylquinoline which had been obtained from 2,5-dimethoxyaniline according to the method of L. Baxter et al. [J.C.S. Perkin I:2374-9 (1973).]

T-7 and T-8 were prepared according to the method of H. Link et al. [Helvetica Chimica Acta, 65:2645 (1982).]

T-10 was purchased from the market (resazurin; No. 19,930-3, Aldrich.)

T-11 was prepared from T-1 and O-aminophenol according to the specification of the Japanese Patent Publication No. 1782/61.

Test methods:

Sample solutions as shown below were aseptically prepared from bovine serum albumine (No. A-8022, Sigma)(hereinafter referred to as BSA), 50 mM phosphate buffer solution (pH 7.3) and the test compounds, T-1 to T-11, and aminoguanidine.

[Sample solutions]

| | |
|---|---|
| Normal sample; | 20 mg/ml BSA in buffer solution |
| Control sample; | 20 mg/ml BSA and 50 mM glucose in buffer solution |
| Test sample; | 20 mg/ml BSA, 50 mM glucose and 5 mM test compound in buffer solution |

The sample solutions were kept for 4 weeks at 37 °C, and the amount of furosine which was formed by non-enzymatic glycosylation was determined by HPLC according to the method of Schleicher et al. [J. Clin. Biochem., 19:81-87 (1981).] Thus, the sample solutions after reaction were dialyzed, and aliquots of 1 ml were lyophylized and then hydrolyzed by the addition of 1 ml of 6 N hydrochloric acid followed by heating at 100 °C for 20 hours. After removal of hydrochloric acid by evaporation, 1 ml of water was added to each sample, and the samples were subjected to filtration using a filter with the pore size of 0.45 μm. The filtrate was used as the sample for HPLC. ODS-120T (Toso) was used for the column and 7 mM phosphoric acid solution was used as the eluant. The absorbance peak whose ratio of peak area at 280 mm/254 mm was 3.9/1 was regarded as the peak corresponding to furosine.

Upon the area of the peak of furosine of each sample, the inhibition rate of furosine formation by the test compound was calculated as follows.

$$\text{Inhibition rate (\%)} = (c-d) \div (c-n) \times 100$$

c; peak area of furosine of the control sample
d; peak area of furosine of the test sample

n;  peak area of furosine of the normal sample

Results:

As shown in Table 1, each test compound, T-1 to T-11, exhibited a remarkably potent inhibitory effect in comparison with aminoguanidine, a known inhibitor of Maillard's reaction.

The following examples of pharmaceutical compositions are offered for illustrating purposes only. Each compound code used therein corresponds to each of the compounds listed in Table 1.

Example 1 Oral tablets

According to the formula below, the ingredients are admixed and formed into tablets by the conventional method. Sugar coating may optionally be made.

| | |
|---|---|
| T-1 | 100 mg |
| lactose | 80 mg |
| corn starch | 17 mg |
| magnesium stearate | 3 mg |

Example 2 Oral tablets

According to the formula below, the ingredients are admixed and formed into tablets by the conventional method. Sugar coating may optionally be made.

| | |
|---|---|
| T-2 | 50 mg |
| Corn starch | 90 mg |
| lactose | 30 mg |
| hydroxypropylcellulose | 25 mg |
| magnesium stearate | 5 mg |

Example 3 Capsules

According to the formula below, the ingredients are admixed, granulated and filled in capsules at an amount of 100 mg/capsule.

| | |
|---|---|
| T-5 | 10 mg |
| corn starch | 45 mg |
| lactose | 20 mg |
| crystalline cellulose | 24 mg |
| talc | 0.5mg |
| magnesium stearate | 0.5mg |

Example 4 Injection

According to the formula below, the ingredients are admixed by the conventional method to dissolve. The solution is filtered, filled into vials and autoclaved to sterilize.

| | |
|---|---|
| T-7 | 20 mg |
| chlorobutanol | 5 mg |
| water for injection | to 1 ml |

Example 5 Eye drops

According to the formula below, the ingredients are admixed by the conventional method to dissolve, and the solution is sterilized by filtration.

| | |
|---|---|
| T-10 | 0.5 g |
| boric acid | 1.0 g |
| borax | q.s. |
| sodium chloride | 0.25 g |
| disodium edetate | 0.02 g |
| chlorobutanol | 0.2 g |
| polysorbate 80 | 0.2 g |
| sodium sulfite | 0.2 g |
| sterile purified water | to 100 ml |

Example 6 Eye ointment

According to the formula below, the ingredients are admixed by the conventional method to form eye ointment.

| | |
|---|---|
| T-11 | 0.5 g |
| white vaseline | 100 g |

Table 1

| Test compound | | Inhibition rate (%) |
|---|---|---|
| T-1 | 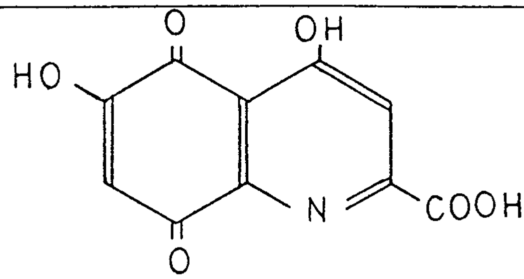 | 77.8 |
| T-2 | 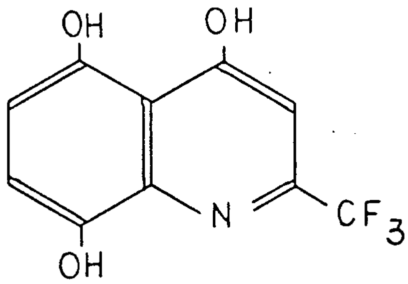 | 79.9 |
| T-3 | 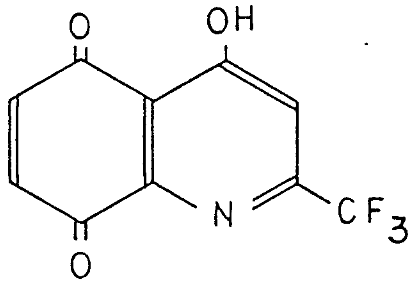 | 73.4 |
| T-4 | 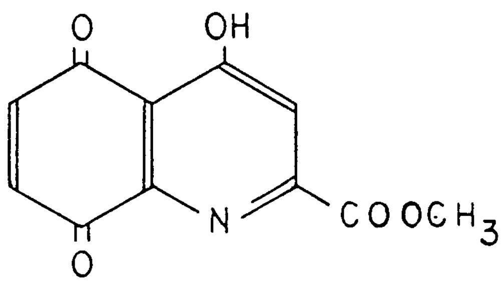 | 92.5 |

Table 1 (Continued)

| | | |
|---|---|---|
| T-5 | 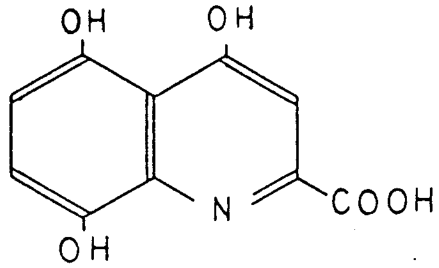 | 78.9 |
| T-6 | 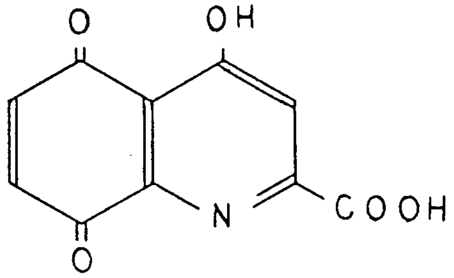 | 76.0 |
| T-7 | 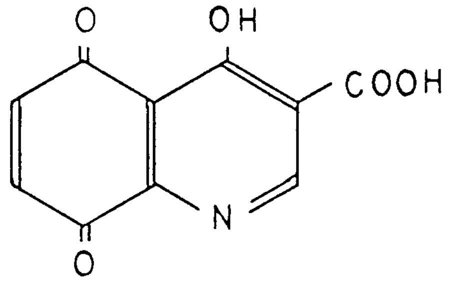 | 79.9 |
| T-8 | 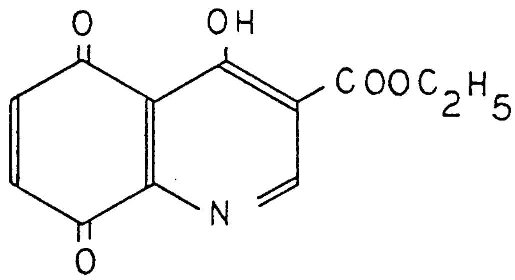 | 96.9 |

Table 1 (Continued)

| | | |
|---|---|---|
| T-9 | 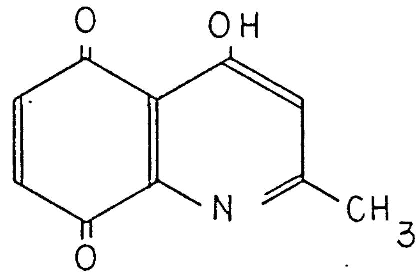 | 99.5 |
| T-10 | 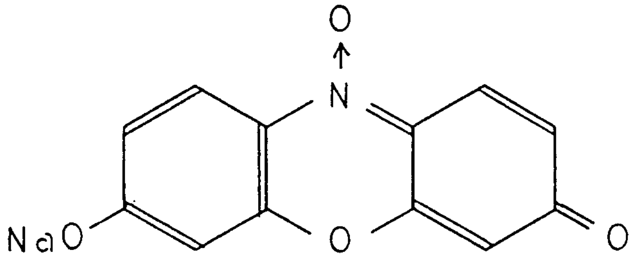 | 73.8 |
| T-11 | 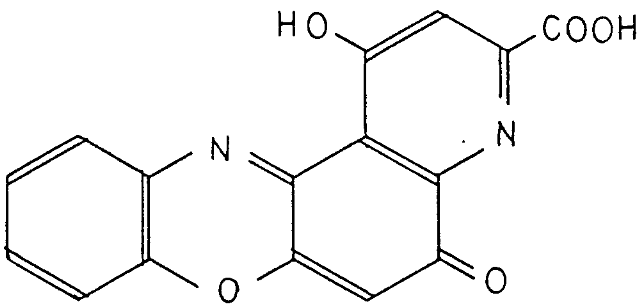 | 96.6 |
| Aminoguanidine | | 7.5 |

## Claims

**Claims for the following Contracting States : AT, BE, CH, DE, DK, FR, GB, IT, LI, LU, NL, SE**

**1.** A pharmaceutical composition comprising in admixture with a pharmaceutically acceptable carrier:

(a) a 4-hydroxy-5,8-dioxoquinoline derivative of the formula (1) or a pharmaceutically acceptable salt thereof:

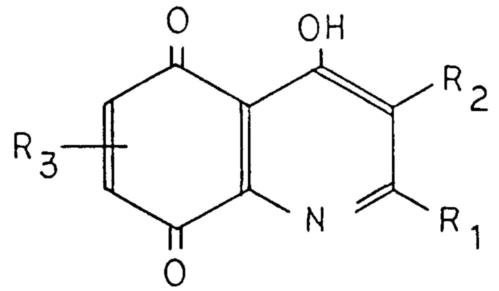

(I)

wherein $R_1$ and $R_2$ are each hydrogen, methyl, trifluoromethyl, carboxy, methoxycarbonyl or ethoxycarbonyl, and $R_3$ is hydrogen or hydroxy;

(b) a 4,5,8-trihydroxyquinoline derivative of the formula (II) or a pharmaceutically acceptable salt thereof:

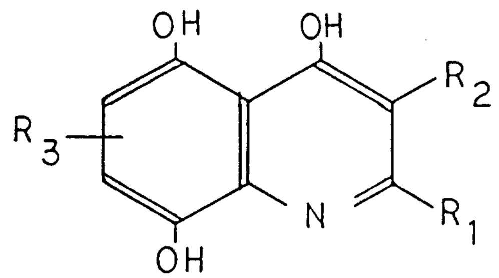

(II)

wherein $R_1$, $R_2$ and $R_3$ are as defined hereinbefore;

(c) a 3-oxophenoxazine derivative of the formula (III) or a pharmaceutically acceptable salt thereof:

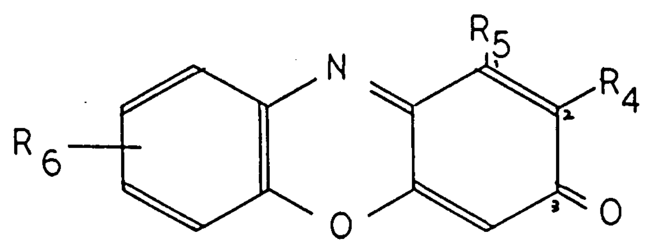

(III)

wherein $R_4$ and $R_5$ are each hydrogen or form, by incorporation of $C_1$ and $C_2$ carbon atoms, a condensed [2,1-b] pyridine ring optionally substituted with a hydroxyl group and/or a carboxyl group, and $R_6$ is hydrogen or hydroxy; or

(d) a 3-oxophenoxazine N-oxide of the formula (IV) or a pharmaceutically acceptable salt thereof:

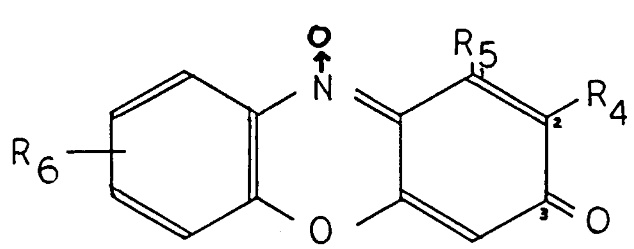

(IV)

wherein $R_4$, $R_5$ and $R_6$ are as defined hereinbefore; with the proviso that resazurin is not applied.

**2.** A pharmaceutical composition of Claim 1 which is in the form of tablets, pills, powder, granules, capsules, injection, eye drops or eye ointment.

**3.** A method of preparing a pharmaceutical composition which comprises admixing with a pharmaceutically acceptable carrier a pharmacologically effective amount of;

(a) a 4-hydroxy-5,8-dioxoquinoline derivative of the formula (I) or a pharmaceutically acceptable salt thereof:

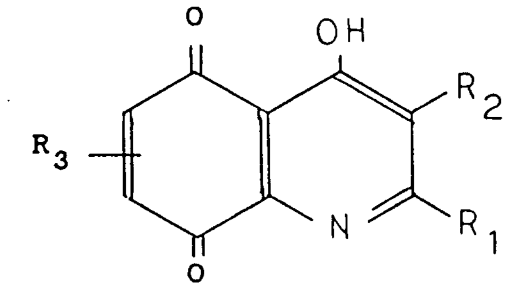

(I)

wherein $R_1$ and $R_2$ are each hydrogen, methyl, trifluoromethyl, carboxy, methoxycarbonyl or ethoxycarbonyl, and $R_3$ is hydrogen or hydroxy;

(b) a 4,5,8-trihydroxy quinoline derivative of the formula (II) or a pharmaceutically acceptable salt thereof:

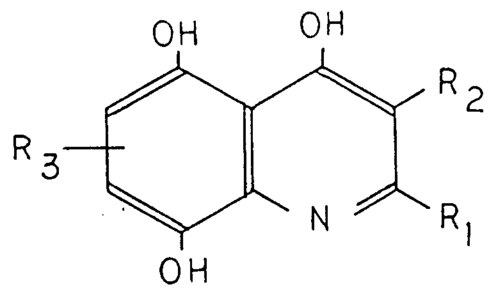

(II)

wherein $R_1$, $R_2$ and $R_3$ are as defined hereinbefore;

(c) a 3-oxophenoxazine derivative of the formula (III) or a pharmaceutically acceptable salt thereof:

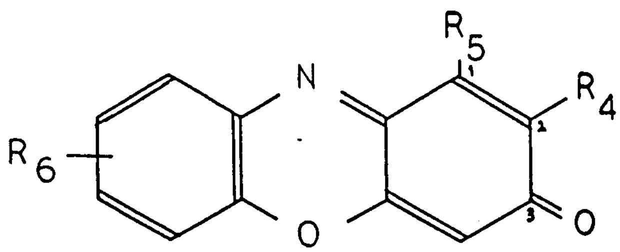

(III)

wherein $R_4$ and $R_5$ are each hydrogen or form, by incorporation of $C_1$ and $C_2$ carbon atoms, a condensed [2,1-b] pyridine ring optionally substituted with a hydroxyl group and/or a carboxyl group, and $R_6$ is hydrogen or hydroxy; or

(d) a 3-oxophenoxazine N-oxide of the formula (IV) or a pharmaceutically acceptable salt thereof:

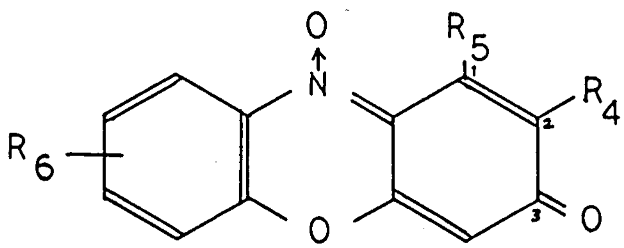

(IV)

wherein $R_4$, $R_5$ and $R_6$ are as defined hereinbefore; with the proviso that resazurin is not applied.

4. A method of Claim 3 wherein the composition is in the form of tablets, pills, powder, granules, capsules, injection, eye drops or eye ointment.

5. The use of the compounds of formula (I), (II), (III) or (IV) as defined in Claim 1 inclusive of resazurin for preparing a pharmaceutical composition for the treatment or prophylaxis of the disorders in human body which may develop via Maillard's reaction in said human body; wherein said disorders are diabetic complications such as coronary heart disease, peripheral circulation disorders, cerebrovascular disorders, neuropathy, nephropathy, arteriosclerosis, arthrosclerosis, cataract and retinopathy, or age-associated disorders such as atherosclerosis, coronary heart disease, cerebrovascular disorders and senile cataract.

**Claims for the following Contracting States : ES, GR**

1. A method of preparing a pharmaceutical composition which comprises admixing with a pharmaceutically acceptable carrier a pharmacologically effective amount of;

(a) a 4-hydroxy-5,8-dioxoquinoline derivative of the formula (I) or a pharmaceutically acceptable salt thereof:

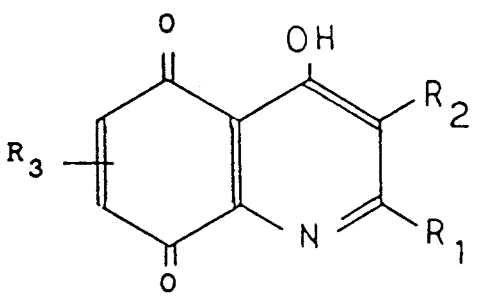

(I)

wherein $R_1$ and $R_2$ are each hydrogen, methyl, trifluoromethyl, carboxy, methoxycarbonyl or ethoxycarbonyl, and $R_3$ is hydrogen or hydroxy;
(b) a 4,5,8-trihydroxy quinoline derivative of the formula (II) or a pharmaceutically acceptable salt thereof:

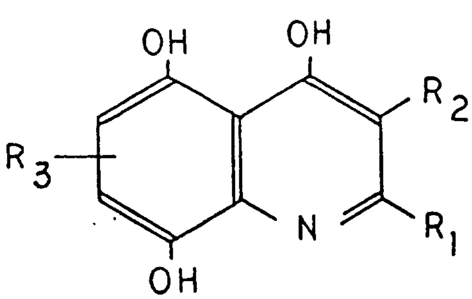

(II)

wherein $R_1$, $R_2$ and $R_3$ are as defined hereinbefore;

(c) a 3-oxophenoxazine derivative of the formula (III) or a pharmaceutically acceptable salt thereof:

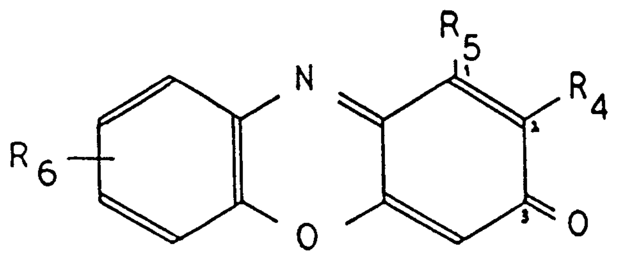

(III)

wherein $R_4$ and $R_5$ are each hydrogen or form, by incorporation of $C_1$ and $C_2$ carbon atoms, a condensed [2,1-b] pyridine ring optionally substituted with a hydroxyl group and/or a carboxyl group, and $R_6$ is hydrogen or hydroxy; or

(d) a 3-oxophenoxazine N-oxide of the formula (IV) or a pharmaceutically acceptable salt thereof:

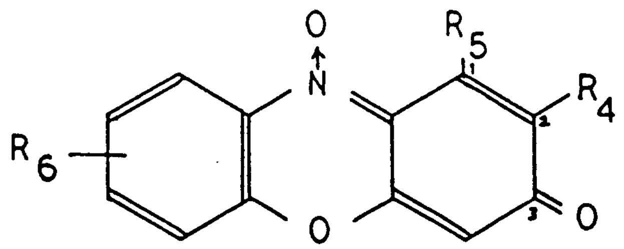

(IV)

wherein $R_4$, $R_5$ and $R_6$ are as defined hereinbefore; with the proviso that resazurin is not applied.

**2.** A method of Claim 1 wherein the composition is in the form of tablets, pills, powder, granules, capsules, injection, eye drops or eye ointment.

**3.** The use of the compounds of formula (I), (II), (III) or (IV) as defined in Claim 1 inclusive of resazurin for preparing a pharmaceutical composition for the treatment or prophylaxis of the disorders in human body which may develop via Maillard's reaction in said human body; wherein said disorders are diabetic complications such as coronary heart disease, peripheral circulation disorders, cerebrovascular disorders, neuropathy, nephropathy, arteriosclerosis, arthrosclerosis, cataract and retinopathy, or age-associated disorders such as atherosclerosis, coronary heart disease, cerebrovascular disorders and senile cataract.

**Patentansprüche**

**Patentansprüche für folgende Vertragsstaaten : AT, BE, CH, DE, DK, FR, GB, IT, LI, LU, NL, SE**

**1.** Pharmazeutisches Mittel, umfassend im Gemisch mit einem pharmazeutisch akzeptablen Träger:

(a) ein 4-Hydroxy-5,8-dioxochinolin-Derivat der Formel (I) oder ein pharmazeutisch akzeptables Salz davon:

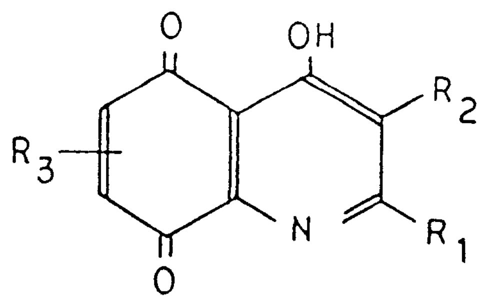

(I)

worin $R_1$ und $R_2$ jeweils für Wasserstoff, Methyl, Trifluormethyl, Carboxy, Methoxycarbonyl oder Ethoxycarbonyl und $R_3$ für Wasserstoff oder Hydroxy stehen;

(b) ein 4,5,8-Trihydroxychinolin-Derivat der Formel (II) oder ein pharmazeutisch akzeptables Salz davon:

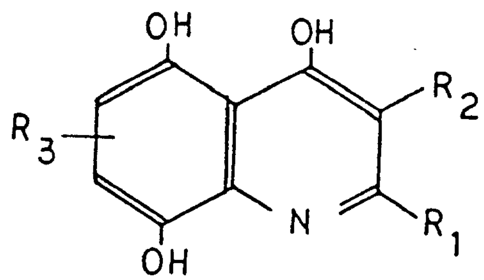

(II)

worin $R_1$, $R_2$ und $R_3$ wie oben definiert sind;

(c) ein 3-Oxophenoxazin-Derivat der Formel (III) oder ein pharmazeutisch akzeptables Salz davon:

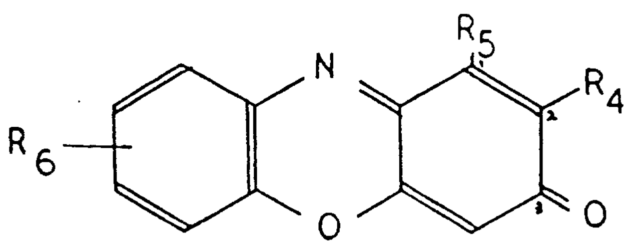

(III)

worin $R_4$ und $R_5$ jeweils für Wasserstoff stehen oder durch Einbindung des $C_1$- und $C_2$-Kohlenstoffatoms einen kondensierten [2,1-b]-Pyridinring bilden, der gegebenenfalls mit einer Hydroxylgruppe und/oder einer Carboxylgruppe substituiert ist, und $R_6$ für Wasserstoff oder Hydroxy steht; oder

(d) ein 3-Oxophenoxazin-N-oxid der Formel (IV) oder ein pharmazeutisch akzeptables Salz davon:

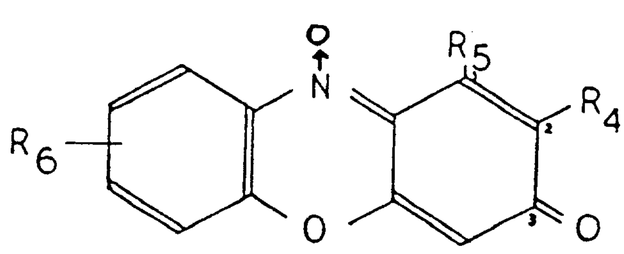

(IV)

worin $R_4$, $R_5$ und $R_6$ wie oben definiert sind; mit der Maßgabe, daß Resazurin nicht verwendet wird.

2. Pharmazeutisches Mittel nach Anspruch 1, das in Tabletten-, Pillen-, Pulver-, Granulat-, Kapsel-, Injektions-, Augentropfen- oder Augensalbenform vorliegt.

3. Verfahren zur Herstellung eines pharmazeutischen Mittels, wobei man eine pharmazeutisch wirksame Menge:

(a) eines 4-Hydroxy-5,8-dioxochinolin-Derivats der Formel (I) oder eines pharmazeutisch akzeptablen Salzes davon:

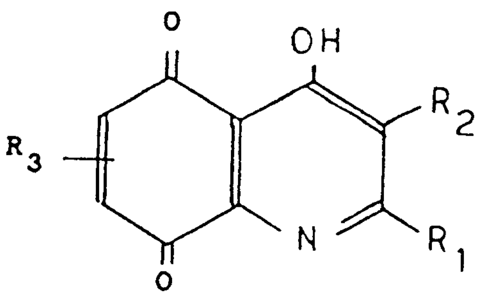
(I)

worin $R_1$ und $R_2$ jeweils für Wasserstoff, Methyl, Trifluormethyl, Carboxy, Methoxycarbonyl oder Ethoxycarbonyl und $R_3$ für Wasserstoff oder Hydroxy stehen;
(b) eines 4,5,8-Trihydroxychinolin-Derivats der Formel (II) oder eines pharmazeutisch akzeptablen Salzes davon:

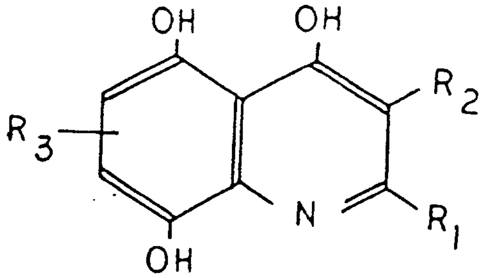
(II)

worin $R_1$, $R_2$ und $R_3$ wie oben definiert sind;
(c) eines 3-Oxophenoxazin-Derivats der Formel (III) oder eines pharmazeutisch akzeptablen Salzes davon:

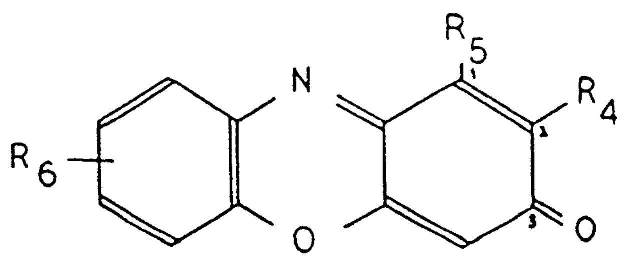
(III)

worin $R_4$ und $R_5$ jeweils für Wasserstoff stehen oder durch Einbindung des $C_1$- und $C_2$-Kohlenstoffatoms einen kondensierten [2,1-b]-Pyridinring bilden, der gegebenenfalls mit einer Hydroxylgruppe und/oder einer Carboxylgruppe substituiert ist, und $R_6$ für Wasserstoff oder Hydroxy steht; oder
(d) eines 3-Oxophenoxazin-N-oxids der Formel (IV) oder eines pharmazeutisch akzeptablen Salzes davon:

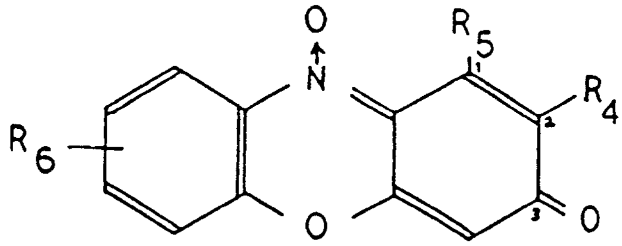

(IV)

worin $R_4$, $R_5$ und $R_6$ wie oben definiert sind; mit der Maßgabe, daß Resazurin nicht verwendet wird, mit einem pharmazeutisch akzeptablen Träger vermischt.

**4.** Verfahren nach Anspruch 3, wobei das Mittel in Tabletten-, Pillen-, Pulver-, Granulat-, Kapsel-, Injektions-, Augentropfen- oder Augensalbenform vorliegt.

**5.** Verwendung der Verbindungen der Formel (I), (II), (III) oder (IV) nach Anspruch 1 einschließlich Resazurin zur Herstellung eines pharmazeutischen Mittels zur Behandlung oder Prophylaxe von Erkrankungen des menschlichen Körpers, die sich über die Maillard's Reaktion im menschlichen Körper entwickeln können; wobei die Erkrankungen diabetische Komplikationen, wie koronare Herzerkrankungen, periphere Durchblutungsstörungen, cerebrovaskuläre Erkrankungen, Neuropathie, Nephropathie, Arteriosklerose, Arthrosklerose, Katarakt und Retinopathie, oder altersbedingte Erkrankungen, wie Atherosklerose, koronare Herzerkrankungen, cerebrovaskuläre Erkrankungen und Alterskatarakt, umfassen.

**Patentansprüche für folgende Vertragsstaaten : ES, GR**

**1.** Verfahren zur Herstellung eines pharmazeutischen Mittels, wobei man eine pharmazeutisch wirksame Menge:

(a) eines 4-Hydroxy-5,8-dioxochinolin-Derivats der Formel (I) oder eines pharmazeutisch akzeptablen Salzes davon:

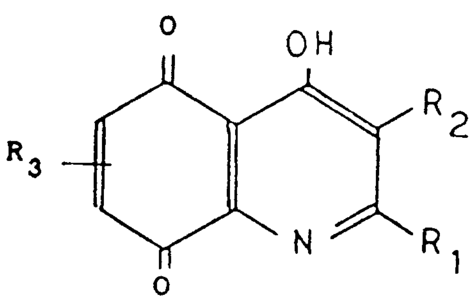

(I)

worin $R_1$ und $R_2$ jeweils für Wasserstoff, Methyl, Trifluormethyl, Carboxy, Methoxycarbonyl oder Ethoxycarbonyl und $R_3$ für Wasserstoff oder Hydroxy stehen;
(b) eines 4,5,8-Trihydroxychinolin-Derivats der Formel (II) oder eines pharmazeutisch akzeptablen Salzes davon:

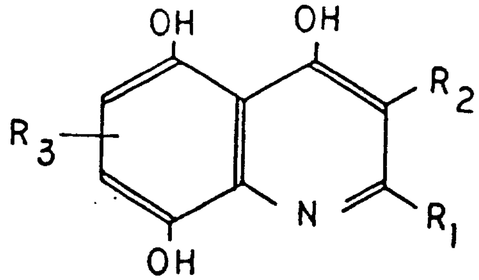

(I)

worin $R_1$, $R_2$ und $R_3$ wie oben definiert sind;

(c) eines 3-Oxophenoxazin-Derivats der Formel (III) oder eines pharmazeutisch akzeptablen Salzes davon:

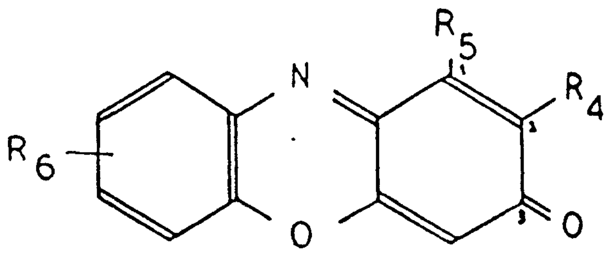

(III)

worin $R_4$ und $R_5$ jeweils für Wasserstoff stehen oder durch Einbindung des $C_1$- und $C_2$-Kohlenstoffatoms einen kondensierten [2,1-b]-Pyridinring bilden, der gegebenenfalls mit einer Hydroxylgruppe und/oder einer Carboxylgruppe substituiert ist, und $R_6$ für Wasserstoff oder Hydroxy steht; oder

(d) eines 3-Oxophenoxazin-N-oxids der Formel (IV) oder eines pharmazeutisch akzeptablen Salzes davon:

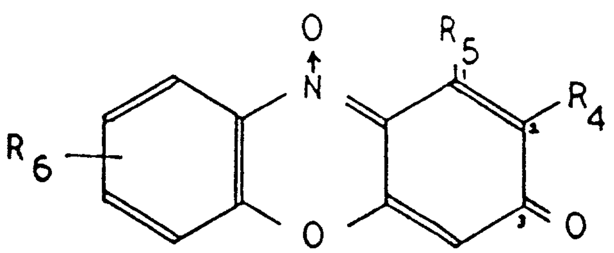

(IV)

worin $R_4$, $R_5$ und $R_6$ wie oben definiert sind; mit der Maßgabe, daß Resazurin nicht verwendet wird, mit einem pharmazeutisch akzeptablen Träger vermischt.

2. Verfahren nach Anspruch 1, wobei das Mittel in Tabletten-, Pillen-, Pulver-, Granulat-, Kapsel-, Injektions-, Augentropfen- oder Augensalbenform vorliegt.

3. Verwendung der Verbindungen der Formel (I), (II), (III) oder (IV) nach Anspruch 1 einschließlich Resazurin zur Herstellung eines pharmazeutischen Mittels zur Behandlung oder Prophylaxe von Erkrankungen des menschlichen Körpers, die sich über Maillard's Reaktion im menschlichen Körper entwickeln können; wobei die Erkrankungen diabetische Komplikationen, wie koronare Herzerkrankungen, periphere Durchblutungsstörungen, cerebrovaskuläre Erkrankungen, Neuropathie, Nephropathie, Arteriosklerose, Arthrosklerose, Katarakt und Retinopathie, oder altersbedingte Erkrankungen, wie Atherosklerose, koronare Herzerkrankungen, cerebrovaskuläre Erkrankungen und Alterskatarakt, umfassen.

**Revendications**

**Revendications pour les Etats contractants suivants : AT, BE, CH, DE, DK, FR, GB, IT, LI, LU, NL, SE**

1. Composition pharmaceutique comprenant en mélange avec un véhicule pharmaceutiquement acceptable de :

(a) un dérivé de 4-hydroxy-5,8-dioxoquinoline présentant la formule (I) ou un sel pharmaceutiquement acceptable de ce dérivé :

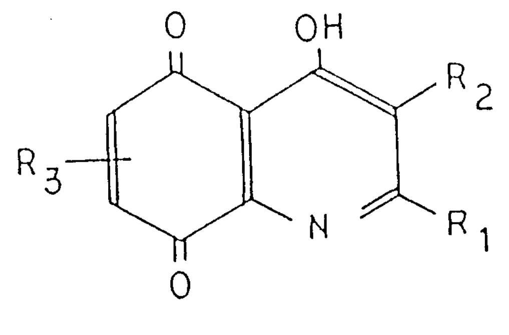

(I)

dans laquelle $R_1$ et $R_2$ sont chacun un atome d'hydrogène, un groupe méthyle, trifluorométhyle, carboxy, méthoxycarbonyle ou éthoxycarbonyle, et $R_3$ est un atome d'hydrogène ou un groupe hydroxy ;

(b) un dérivé de 4,5,8-trihydroxy-quinoline présentant la formule (II) ou un sel pharmaceutiquement acceptable de ce dérivé :

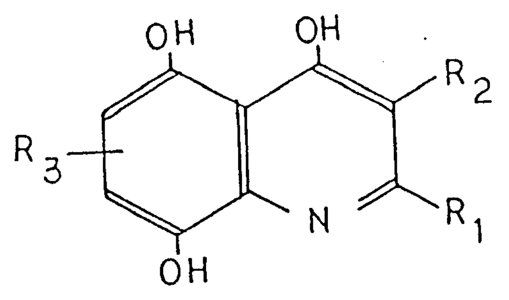

(II)

dans laquelle $R_1$, $R_2$ et $R_3$ sont tels que définis ci-dessus ;

(c) un dérivé de 3-oxophénoxazine présentant la formule (III) ou un sel pharmaceutiquement acceptable de ce dérivé :

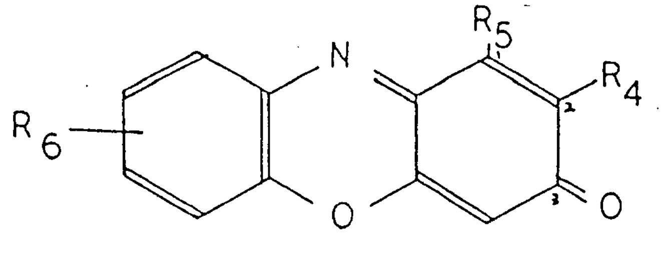

(III)

dans laquelle $R_4$ et $R_5$ sont chacun un atome d'hydrogène ou forment ensemble, par incorporation d'atomes de carbone C1 et C2, un noyau condensé de [2,1-b] pyridine, éventuellement substitué par un groupe hydroxyle et/ou un groupe carboxyle, et $R_6$ est un atome d'hydrogène ou un groupe hydroxy ;

(d) ou un N-oxyde de 3-oxophénoxazine présentant la formule (IV) ou un sel pharmaceutiquement acceptable de ce dérivé :

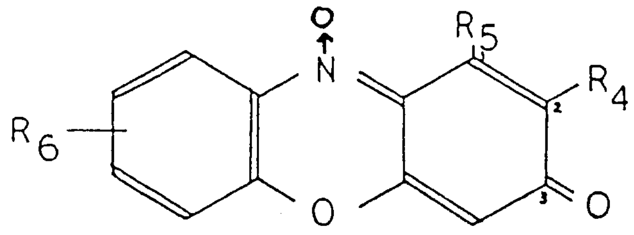

(IV)

où $R_4$, $R_5$ et $R_6$ sont tels que définis ci-dessus ; sous la réserve qu'il ne s'agisse pas de résazurine.

**2.** Composition pharmaceutique selon la revendication 1 présentée sous forme de comprimés, pilules, poudre, granulés, gélules, solution injectable, gouttes oculaires ou crème oculaire.

**3.** Procédé de préparation d'une composition pharmaceutique consistant à mélanger avec un véhicule pharmaceutiquement acceptable, une préparation pharmacologiquement active de :

(a) un dérivé de 4-hydroxy-5,8-dioxoquinoline présentant la formule (I) ou un sel pharmaceutiquement acceptable de ce dérivé :

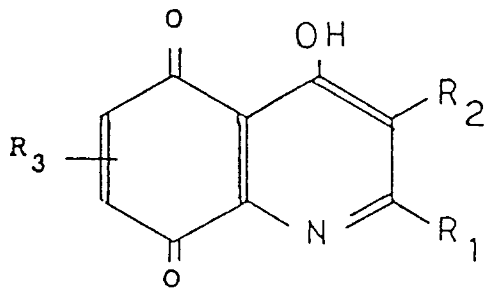

(I)

dans laquelle $R_1$ et $R_2$ sont chacun un atome d'hydrogène, un groupe méthyle, trifluorométhyle, carboxy, méthoxycarbonyle ou éthoxycarbonyle, et $R_3$ est un atome d'hydrogène ou un groupe hydroxy ;

(b) un dérivé de 4,5,8-trihydroxy-quinoline présentant la formule (II) ou un sel pharmaceutiquement acceptable de ce dérivé :

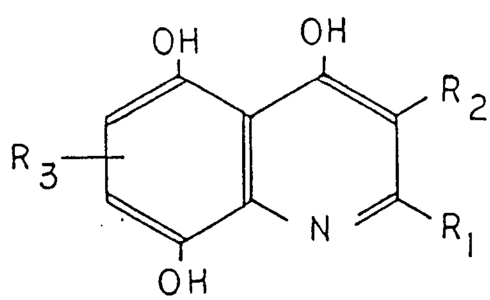

(II)

dans laquelle $R_1$, $R_2$ et $R_3$ sont tels que définis ci-dessus ;

(c) un dérivé de 3-oxophénoxazine présentant la formule (III) ou un sel pharmaceutiquement acceptable de ce dérivé :

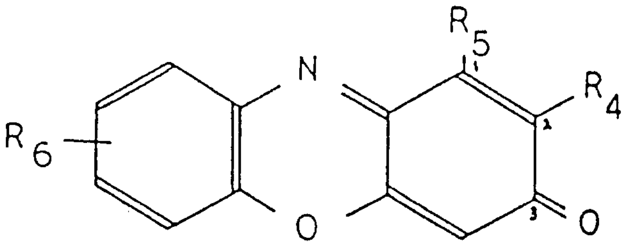

(III)

dans laquelle $R_4$ et $R_5$ sont chacun un atome d'hydrogène ou forment ensemble, par incorporation d'atomes de carbone C1 et C2, un noyau condensé de [2,1-b] pyridine, éventuellement substitué par un groupe hydroxyle et/ou un groupe carboxyle, et $R_6$ est un atome d'hydrogène ou un groupe hydroxy ;

(d) ou un N-oxyde de 3-oxophénoxazine présentant la formule (IV) ou un sel pharmaceutiquement acceptable de ce dérivé :

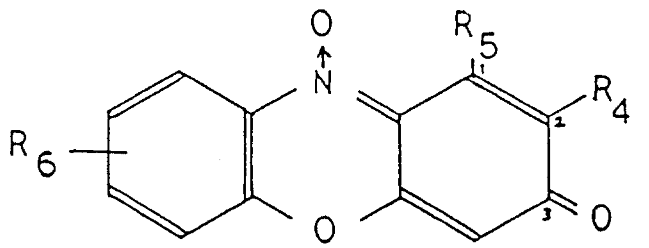

(IV)

où $R_4$, $R_5$ et $R_6$ sont tels que définis ci-dessus ; sous la réserve qu'il ne s'agisse pas de résazurine.

**4.** Procédé selon la revendication 3 suivant lequel la composition est présentée sous forme de comprimés, pilules, poudre, granulés, gélules, solution injectable, gouttes oculaires ou crème oculaire.

**5.** Application des composés suivant les formules (I), (II), (III) ou (IV) tels que définis dans la revendication 1, y compris la résazurine, à la préparation d'une composition pharmaceutique pour le traitement ou la prévention des pathologies humaines qui se développent dans le corps humain par l'intermédiaire de la réaction de Maillard, lesdites pathologies étant les complications du diabète telles que les maladies cardiaques coronariennes, les troubles circulatoires périphériques, les maladies cérébro-vasculaires, les neuropathies, les néphropathies, l'artérosclérose, l'artrosclérose, la cataracte et la rétinopathie, ou les maladies de la vieillesse telles que l'atérosclérose, les maladies cardiaques coronariennes, les troubles cérébrovasculaires et la cataracte sénile.

**Revendications pour les Etats contractants suivants : ES, GR**

**1.** Procédé de préparation d'une composition pharmaceutique comprenant en mélange avec un véhicule pharmaceutiquement acceptable une préparation pharmacologiquement active de :

(a) un dérivé de 4-hydroxy-5,8-dioxoquinoline présentant la formule (I) ou un sel pharmaceutiquement acceptable de ce dérivé :

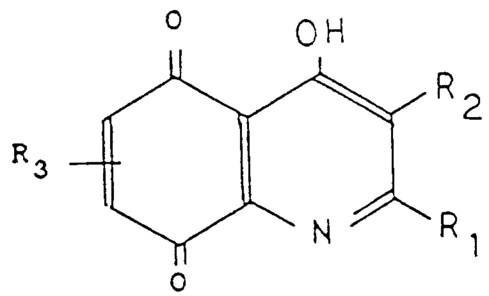

(I)

dans laquelle $R_1$ et $R_2$ sont chacun un atome d'hydrogène, un groupe méthyle, trifluorométhyle, carboxy, méthoxycarbonyle ou éthoxycarbonyle, et $R_3$ est un atome d'hydrogène ou un groupe hydroxy ;

(b) un dérivé de 4,5,8-trihydroxy-quinoline présentant la formule (II) ou un sel pharmaceutiquement acceptable de ce dérivé :

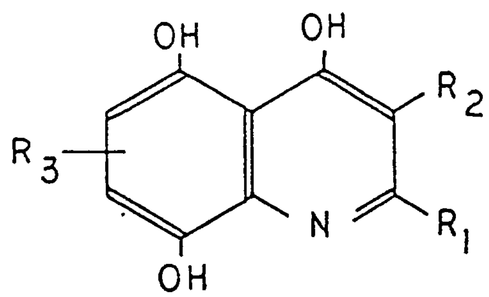

(II)

dans laquelle $R_1$, $R_2$ et $R_3$ sont tels que définis ci-dessus ;

(c) un dérivé de 3-oxophénoxazine présentant la formule (III) ou un sel pharmaceutiquement acceptable de ce dérivé :

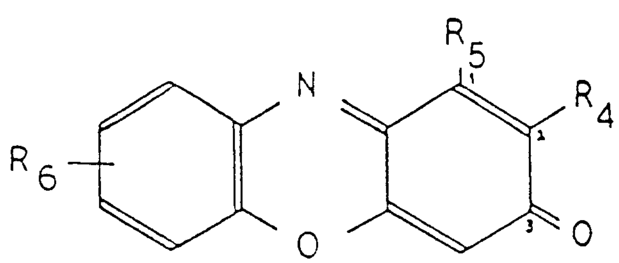

(III)

dans laquelle $R_4$ et $R_5$ sont chacun un atome d'hydrogène ou forment ensemble, par incorporation d'atomes de carbone C1 et C2, un noyau condensé de [2,1-b] pyridine, éventuellement substitué par un groupe hydroxyle et/ou un groupe carboxyle, et $R_6$ est un atome d'hydrogène ou un groupe hydroxy ;

(d) ou un N-oxyde de 3-oxophénoxazine présentant la formule (IV) ou un sel pharmaceutiquement acceptable de ce dérivé :

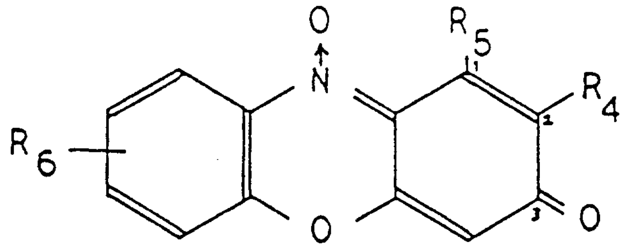

(IV)

où $R_4$, $R_5$ et $R_6$ sont tels que définis ci-dessus ; sous la réserve qu'il ne s'agisse pas de résazurine.

2. Procédé selon la revendication 1 suivant lequel la composition est présentée sous forme de comprimés, pilules, poudre, granulés, gélules, solution injectable, gouttes oculaires ou crème oculaire.

3. Application des composés suivant les formules (I), (II), (III) ou (IV) tels que définis dans la revendication 1, y compris la résazurine, à la préparation d'une composition pharmaceutique pour le traitement ou la prévention des pathologies humaines qui se développent dans le corps humain par l'intermédiaire de la réaction de Maillard, lesdites pathologies étant les complications du diabète telles que les maladies cardiaques coronariennes, les troubles circulatoires périphériques, les maladies cérébro-vasculaires, les neuropathies, les néphropathies, l'artérosclérose, l'artrosclérose, la cataracte et la rétinopathie, ou les maladies de la vieillesse telles que l'atérosclérose, les maladies cardiaques coronariennes, les troubles cérébrovasculaires et la cataracte sénile.